# EUROPEAN PATENT APPLICATION

(11) **EP 4 318 490 A1**
(43) Date of publication of application: **07.02.2024**
(21) Application number: 21934869.5
(22) Date of filing: 30.03.2021
(51) Int. Cl.: G16H 50/20

(54) **DIAGNOSIS PREDICTION SYSTEM USING DIGITAL DEVICE, LEARNING DEVICE, COMPUTER PROGRAM, DIAGNOSIS PREDICTION METHOD, AND PREDICTION MODEL GENERATION/UPDATING METHOD**

(71) Applicant: Medicolab Co., Ltd., Nagoya-shi Aichi 450-6321 (JP)
(72) Inventor: IKUMI, Kazuhiro, Nagoya-shi Aichi 450-6321 (JP)
(74) Representative: V.O.
(86) International application number: PCT/JP2021/013742
(87) International publication number: WO 2022/208700

(57) **Abstract**

Provided is a diagnosis prediction system capable of predicting specific disease-related information. A diagnosis prediction system (100) comprises: an input unit (11) for inputting biological data measured by a digital device worn by a subject, and medical interview data for the subject; and a prediction unit (12) that uses, as input data, biological data measured by digital devices worn respectively by a plurality of subjects and medical interview data corresponding to the biological data of the plurality of subjects, and by using a trained model that has undergone deep learning using disease-related information corresponding to each of the subjects as teaching data, predicts disease-related information for the subject on the basis of the biological data and the medical interview data for the subject input from the input unit.

## Description

### Technical Field

The present disclosure relates to disease determination and disease management after onset, and technology for predicting disease before onset.

### Background Art

There has been known a technology related to a device that assists physicians in diagnosing diseases and enables highly accurate diagnosis of diseases by reflecting the vital signs, symptoms, medical history of the subject, and the like (see, e.g., Patent Document 1).

### Prior Art Document

### Patent Document

Patent Document 1: Japanese Unexamined Patent Application Publication No. 2017-131495

### Summary of the Invention

### Problems to be Solved by the Invention

The conventional technology as described above can be effectively utilized for those who have developed a disease and have already undergone a medical examination at a specialized medical institution. On the other hand, the conventional technology cannot be effectively utilized for those who have developed a disease but have not yet visited a specialized medical institution or for those who have not yet developed a disease.

It is therefore an object of the present invention to provide a technology that enables disease determination and disease management for a person prior to a visit to a specialized medical institution and prediction of a disease in a person prior to the onset of the disease. Other objects of the invention include providing a diagnosis prediction system that can predict specific disease-related information using a digital device, a learning device, a computer program, a diagnosis prediction method, and a prediction model generation/updating method.

### Means for Solving the Problems

In order to solve the objects mentioned above, according to a first aspect of the invention, a diagnosis prediction system includes: an input unit that receives biological data measured by a digital device worn by a subject and medical questionnaire data of the subject; and a prediction unit that predicts disease-related information of the subject based on the biological data and the medical questionnaire data of the subject received through the input unit by using a trained model generated by deep learning (or machine learning), in which biological data measured by a digital device worn by each of a plurality of subjects and medical questionnaire data corresponding to the biological data of the subjects are used as input data, and disease-related information corresponding to each of the subjects is used as training data.

The disease-related information includes at least one of the following: test score, diagnosis name, time of onset, and clinical rating scale.

According to a second aspect of the invention, a learning device includes: a memory that stores biological data measured by a digital device worn by each of a plurality of subjects, medical questionnaire data corresponding to the biological data of the subjects, and disease-related information corresponding to each of the subjects; and a learning unit that generates a trained model using deep learning, in which the biological data and the medical questionnaire data of the subjects stored in the memory are used as input data, and the disease-related information corresponding to each of the subjects stored in the memory is used as training data.

According to a third aspect of the invention, a computer program, which is executed on an information processing device, causes the information processing device to perform: an input step of receiving biological data measured by a digital device worn by a subject and medical questionnaire data of the subject; and a prediction step of predicting disease-related information of the subject based on the biological data and the medical questionnaire data of the subject received in the input step by using a trained model that has undergone deep learning, in which biological data measured by a digital device worn by each of a plurality of subjects and medical questionnaire data corresponding to the biological data of the subjects are used as input data, and disease-related information corresponding to each of the subjects is used as training data.

According to a fourth aspect of the invention, a diagnosis prediction method, which is implemented by an information processing device, includes: an input step of receiving biological data measured by a digital device worn by a subject and medical questionnaire data of the subject; and a prediction step of predicting disease-related information of the subject based on the biological data and the medical questionnaire data of the subject received in the input step by using a trained model generated by deep learning (or machine learning), in which biological data measured by a digital device worn by each of a plurality of subjects and medical questionnaire data corresponding to the biological data of the subjects are used as input data, and disease-related information corresponding to each of the subjects is used as training data.

According to a fifth aspect of the invention, a prediction model generation/updating method includes a memory step of storing biological data measured by a digital device worn by each of a plurality of subjects, medical questionnaire data corresponding to the biological data of the subjects, and disease-related information corresponding to each of the subjects; and a generation/update step of generating and updating a trained prediction model using deep learning (or machine learning), in which the biological data and the medical questionnaire data of the subjects stored in the memory step are used as input data, and the disease-related information corresponding to each of the subjects stored in the memory step is used as training data.

### Effects of the Invention

According to the present disclosure, it is possible to provide a diagnosis prediction system that can predict specific disease-related information, a learning device, a computer program, a diagnosis prediction method, and a prediction model generation/updating method.

### Brief Description of the Drawings

[FIG. 1] FIG. 1 is a diagram illustrating an example of a data collection system configuration of a diagnosis prediction system according to an embodiment of the invention.
[FIG. 2] FIG. 2 is a diagram illustrating an example of data collection by the diagnosis prediction system according to an embodiment of the invention.
[FIG. 3] FIG. 3 is a diagram illustrating the flow of operations of an examiner system according to an embodiment of the invention.
[FIG. 4] FIG. 4 is a diagram illustrating an example of a screen displayed at the startup of an EDC system according to an embodiment of the invention.
[FIG. 5] FIG. 5 is a diagram illustrating an example of a screen for newly registering a subject in the EDC system.
[FIG. 6] FIG. 6 is a diagram illustrating an example of a screen displayed when a QR code is issued.
[FIG. 7] FIG. 7 is a diagram illustrating an example of data of medical questionnaires for an examiner to ask according to an embodiment of the invention.
[FIG. 8] FIG. 8 is a diagram illustrating an example of a screen displayed at the startup of a dedicated application on an examiner terminal according to an embodiment of the invention.
[FIG. 9] FIG. 9 is a diagram illustrating an example of a device connection between a master device system and a slave device system during measurement according to an embodiment of the invention.
[FIG. 10] FIG. 10 is a diagram illustrating measurement processes according to an embodiment of the invention.
[FIG. 11] FIG. 11 is a diagram illustrating an example of the measurement processes according to an embodiment of the invention.
[FIG. 12] FIG. 12 is a diagram illustrating an example of a table of biological data collected in each process.
[FIG. 13] FIG. 13 is a diagram illustrating an example of data of medical questionnaires for a subject to answer according to an embodiment of the invention.
[FIG. 14] FIG. 14 is a diagram illustrating an example of data collection according to an embodiment of the invention.
[FIG. 15] FIG. 15 is an explanatory diagram illustrating a prediction model generated by a server according to an embodiment of the invention.
[FIG. 16] FIG. 16 is a diagram illustrating features extracted from biological data.
[FIG. 17] FIG. 17 is a diagram illustrating features extracted from biological data.
[FIG. 18] FIG. 18 is a diagram illustrating a model structure according to an embodiment of the invention.
[FIG. 19] FIG. 19 is a diagram illustrating the evaluation of diagnosis name prediction results for the verification/test data of 6 cases.
[FIG. 20] FIG. 20 is a graph illustrating the ROC curves of diagnosis name prediction results in the diagnosis prediction system according to an embodiment of the invention.
[FIG. 21] FIG. 21 is a graph illustrating the distribution of true values and predicted values obtained by the prediction system according to an embodiment of the invention.
[FIG. 22] FIG. 22 is a graph illustrating the distribution of the mean absolute errors of 63 items of the diagnosis prediction result in the test data of 2 cases.

### Modes for Carrying Out the Invention

Hereinafter, exemplary embodiments of the disclosure will be described in detail.

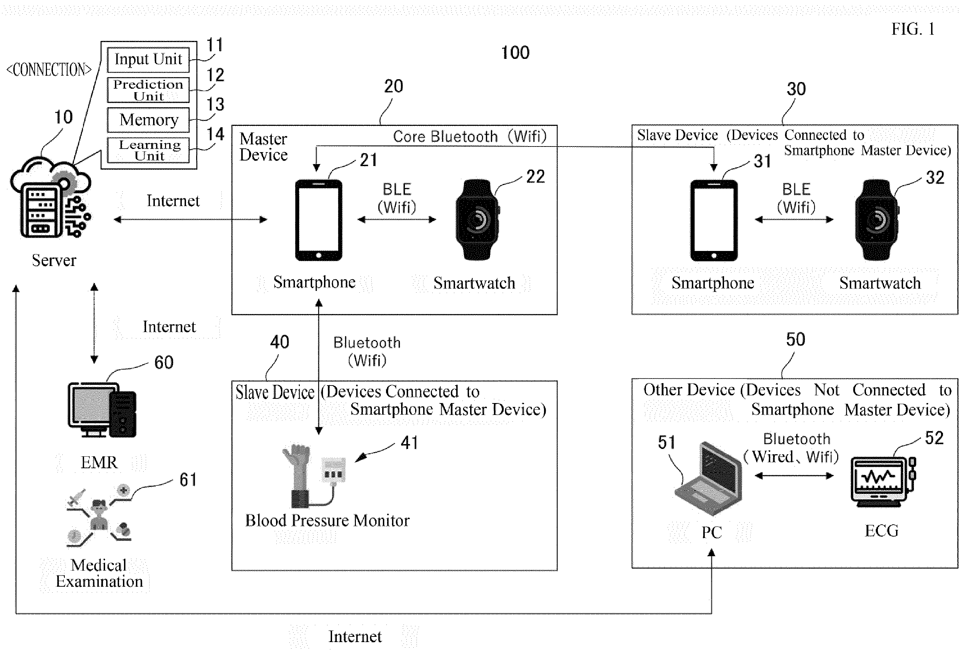

FIG. 1 is a diagram illustrating an example of a system configuration of a diagnosis prediction system according to an embodiment of the invention. As illustrated in FIG. 1, a diagnosis prediction system 100 includes a server 10, an electronic medical record (EMR) system 60, a medical examination system 61, a master device system 20, a slave device system 30, a slave device 40, and other devices 50.

In the diagnosis prediction system 100, a plurality of digital devices are connected via wireless communication, such as Bluetooth (registered trademark) or Wi-Fi (registered trademark), to allow an examiner to efficiently collect medical questionnaire data and biological data from a subject and predict disease-related information.

The disease-related information described herein includes four items: test score, diagnosis name, time of onset, and clinical rating scale. Regarding the clinical rating scale, the aim is to predict existing indicators as well as to construct a new clinical rating scale.

The digital devices are terminals with various built-in sensors or the like for measuring various types of biological or biometric information, such as smartphones, smartwatches, or smart clothes. Each subject wears one or more digital devices. In this embodiment, the digital devices include a smartphone master device 21, a smartphone slave device 31, a smartwatch master device 22, a smartwatch slave device 32, and a blood pressure monitor or electrocardiograph (ECG) 52.

Examples of diagnosis prediction targets include, but are not limited to, neurological diseases such as Parkinson's disease, multiple system atrophy, progressive supranuclear palsy, corticobasal degeneration, Alzheimer's dementia, vascular dementia, Lewy body dementia, multiple sclerosis, and amyotrophic lateral sclerosis.

In this embodiment, an example will be described in which the server 10 predicts disease-related information based on medical questionnaire data and biological data. The server 10 includes one or more servers. The server 10 also functions as a learning device.

The server 10 includes an input unit 11, a prediction unit 12, a memory 13, and a learning unit 14. The server 10 further includes an input/output interface (I/F) to which output devices such as a display monitor and input devices such as a keyboard are connected, and a communication I/F that controls communication using Internet communication via a wireless local area network (LAN), wired LAN, or the like.

The prediction unit 12 includes a central processing unit (CPU), a random access memory (RAM), and a read only memory (ROM). The ROM stores programs executed by the CPU. The RAM is used as a work memory for the CPU. Various programs can be executed by the CPU to cause the prediction unit 12 to perform various operations on the server.

The electronic medical record system 60 includes a server computer and the like and collects electronic medical record information recorded and stored in the electronic medical records of medical institutions that are affiliated with the server 10.

The electronic medical record information refers to the information contained in the electronic medical record (EMR) for each patient, which includes at least patient information related to the patient and medical examination/treatment information related to each medical examination/treatment (start date and time of the examination, disease name, medical questionnaires/examination details, clinical test, image test, photos/videos, etc.).

The medical examination system 61 includes a server computer and the like and collects medical examination information at medical examination institutions that are affiliated with the server 10. The collected medical examination information is registered in association with the attributes (name, date of birth, address, telephone number, insurance card number, medical record ID, etc.) of each user (subject).

The medical examination system 61 manages the medical examination information of users registered in the diagnosis prediction system 100 and the medical examination information of unregistered users in an identifiable manner. In addition, the medical examination system 61 sends the medical examination information of a registered user to the server 10 in response to a request from the server 10.

Note that the electronic medical record system 60 and the medical examination system 61 are not essential elements in the system configuration of the diagnosis prediction system 100 of the embodiment.

The master device system 20 is operated by an examiner and includes the smartphone master device (examiner terminal) 21 and the smartwatch master device 22. The slave device system 30 includes the smartphone slave device (subject terminal) 31 connected to the smartphone master device 21 and the smartwatch slave device 32.

The slave device 40 is a device that is connected to the smartphone master device 21. In this example, the slave device 40 is a blood pressure monitor 41. The slave device 40 may be a device other than the blood pressure monitor 41, and examples thereof include any device as long as it is connected to the smartphone master device 21. The other devices 50 are devices that are not directly connected to the smartphone master device 21 and include a personal computer (PC) 51 and, for example, the electrocardiograph 52.

The smartphone master device 21 or the smartphone slave device 31 collects biological data and medical questionnaire data through a digital device.

Instead of a smartphone, a tablet terminal or a personal computer can be used to replace the smartphone master device 21 or the smartphone slave device 31.

The smartphone master device 21, the smartphone slave device 31, the personal computer 51, or a digital device connected thereto includes a terminal controller, a terminal memory, a camera, an input unit such as a touch pad, a display unit, and a communication I/F that controls communication using Internet communication via a wireless LAN or a cellular telephone network, short-range wireless communication, and the like, which are electrically connected by a communication bus.

The terminal controller includes a CPU, RAM, and ROM. The ROM stores programs executed by the CPU. The RAM is used as a work memory for the CPU. Various programs can be executed by the CPU to cause the terminal controller to implement various functions.

In the diagnosis prediction system 100, the smartphone master device 21 is connected to each digital device via wireless communication, such as Bluetooth (registered trademark) or Wi-Fi (registered trademark), and medical questionnaire data and biological data are efficiently collected.

The data collected on the smartphone master device 21 is sent via the Internet to the server 10 to be stored therein. The smartphone slave device 31, the smartwatch slave device 32 connected thereto, and the blood pressure monitor 41 are illustrated as devices connected to the smartphone master device 21.

Devices other than those connected to the smartphone master device 21, i.e., the other device(s) 50 not connected to the smartphone master device 21, are connected to a dedicated terminal, such as the personal computer 51, via wireless communication to perform data collection. The collected data is temporarily stored in the personal computer 51 and then sent via the Internet to the server 10 to be stored therein.

There may be cases where there is no other device 50 that is not connected to the smartphone master device 21. In such a case, the functions of the smartphone master device 21 or a device connected thereto replace the functions of the other devices.

Data obtained from the electronic medical record system 60, the medical examination system 61, and the like are also stored in the server 10 as necessary.

The server 10 has a diagnosis prediction function and receives biological data measured by a digital device worn by a subject and medical questionnaire data of the subject entered through the input unit 11. The prediction unit 12 uses biological data measured by a digital device worn by each of a plurality of subjects and medical questionnaire data corresponding to the biological data of the subjects as input data and disease-related information corresponding to each of the subjects as training data. The prediction unit 12 predicts disease-related information of the subject based on the biological data and medical questionnaire data of the subject entered from the input unit 11 by using a trained model that has undergone deep learning based on the input data and the training data.

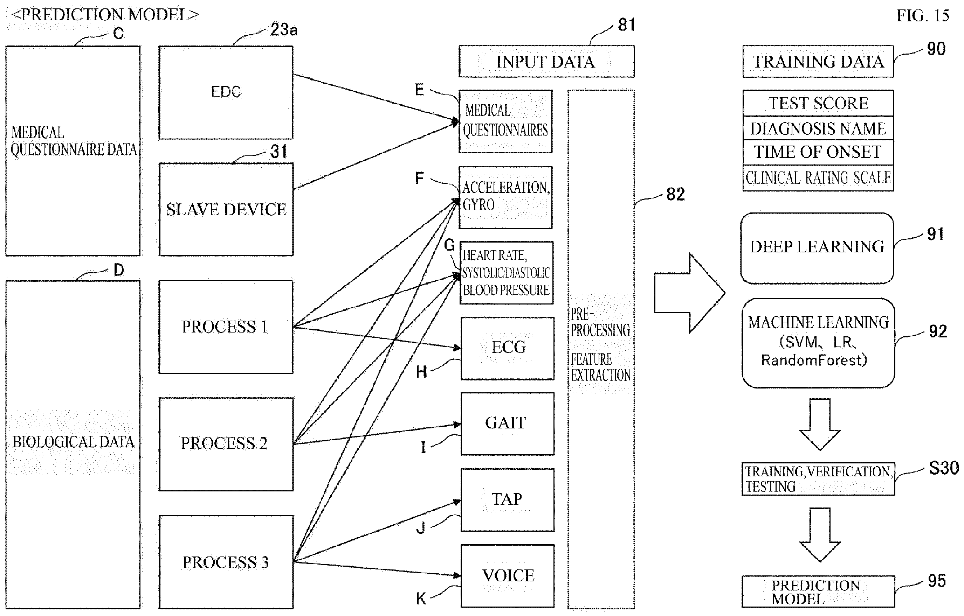

As described above, the server 10 also functions as a learning device. The memory 13 stores biological data measured by a digital device worn by each of a plurality of subjects, medical questionnaire data corresponding to the biological data of the subjects, and disease-related information corresponding to each of the subjects, such as test scores, diagnosis name, time of onset, or clinical rating scale. The learning unit 14 generates a trained prediction model 95 using deep learning 91 (or machine learning), in which the biological data and medical questionnaire data of the subjects stored in the memory 13 are used as input data 81 and the disease-related information corresponding to each of the subjects, such as test scores, diagnosis name, time of onset, or clinical rating scale, stored in the memory 13 is used as training data 90 (see FIG. 15 referred to below).

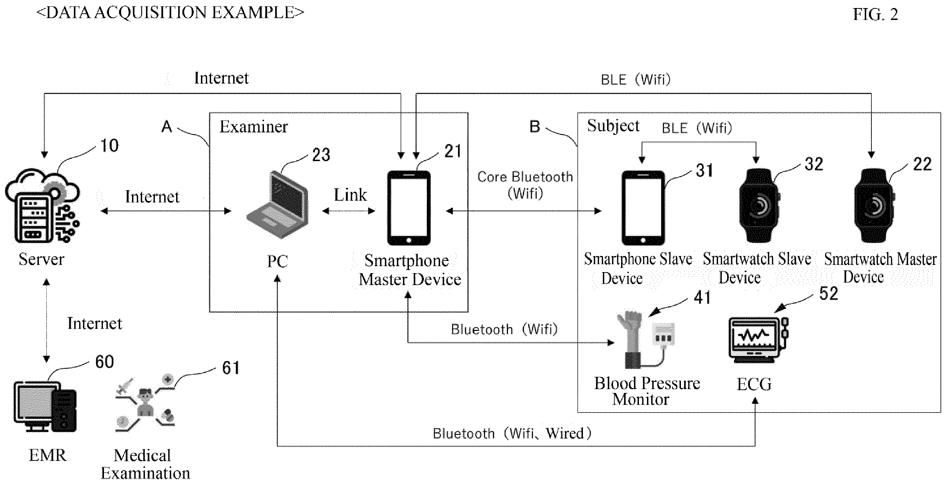

FIG. 2 is a diagram illustrating an example of data collection by the diagnosis prediction system according to an embodiment of the invention. In FIG. 2, an examiner system A is managed by an examiner and includes the smartphone master device 21 and a personal computer 23. Meanwhile, a subject system B is managed by a subject and includes the smartphone slave device 31 and digital devices such as the smartwatch slave device 32, the smartwatch master device 22, the blood pressure monitor 41, and the electrocardiograph 52.

During data collection, the examiner uses the smartphone master device 21 to control devices connected thereto via wireless communication. The other devices that are not connected to the smartphone master device 21, such as the electrocardiograph 52, are connected to and controlled by a terminal, such as the personal computer 23, used by the examiner.

Incidentally, the medical questionnaire data obtained by the examiner from the subject can be entered into the system of the terminal (EDC, described later) to be collected.

The subject uses or wears devices that are connected to and controlled by the smartphone master device 21 (in this example, the smartphone slave device 31, the smartwatch slave device 32, the smartwatch master device 22, and the blood pressure monitor 41) and the other device (s) 50 that is not connected to the smartphone master device 21 (in this example, the electrocardiograph 52), whereby medical questionnaire data and biological data are collected.

There may be cases where there is no other device that is not connected to the smartphone master device 21. In such a case, the functions of the smartphone master device 21 or a device connected thereto replace the functions of the other device.

Data obtained from the electronic medical record system 60, the medical examination system 61, and the like are also stored in the server 10 as necessary.

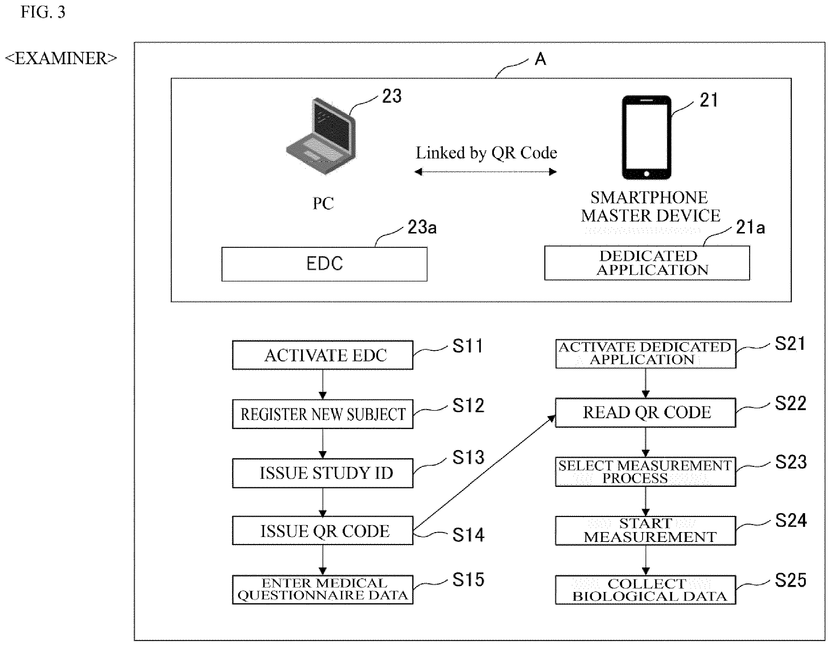

In the following, the flow of operations will be described for both the examiner and the subject. FIG. 3 is a diagram illustrating the flow of operations of the examiner system according to an embodiment of the invention.

The personal computer (information processing device) 23 includes an electronic data capture (EDC) system 23a.

First, the flow of operations performed by the examiner will be described.

In the examiner system A, the EDC system 23a is activated on the personal computer 23 (step S11), and a new subject whose data is to be collected is registered (step S12). Upon completion of the registration of the new subject, the EDC system 23a issues a study ID associated with the subject (step S13). At the start of measurement, the EDC system 23a issues a QR code (code information) containing measurement-related information of the subject (step S11) .

A dedicated application 21a is activated on the smartphone master device 21 (step S21), and the QR code issued by the EDC system 23a is read by the dedicated application 21a on the smartphone master device 21 (step S22). The subject and the measurement-related information are thereby authenticated by the diagnosis prediction system 100.

A measurement process is then selected using the selection means of the smartphone master device 21 (step S23), and the measurement can be started (step S24).

There are prepared a plurality of measurement processes, each of which specifies the selection of digital devices, subject movements, data to be collected, and the like for a series of measurements. The measurement processes each include a plurality of tasks. The diagnosis prediction system 100 collects biological data through a plurality of digital devices according to a sequence of tasks based on the selected measurement process.

Although, in principle, the examiner asks the subject medical questionnaires and enters the data into the EDC system 23a, data of some medical questionnaires is entered directly by the subject into the smartphone slave device 31 to be collected (step S15).

On the other hand, biological data is measured and collected from the subject under the supervision of the examiner according to instructions from the smartphone master device 21, which are defined with respect to each of the measurement processes (step S25).

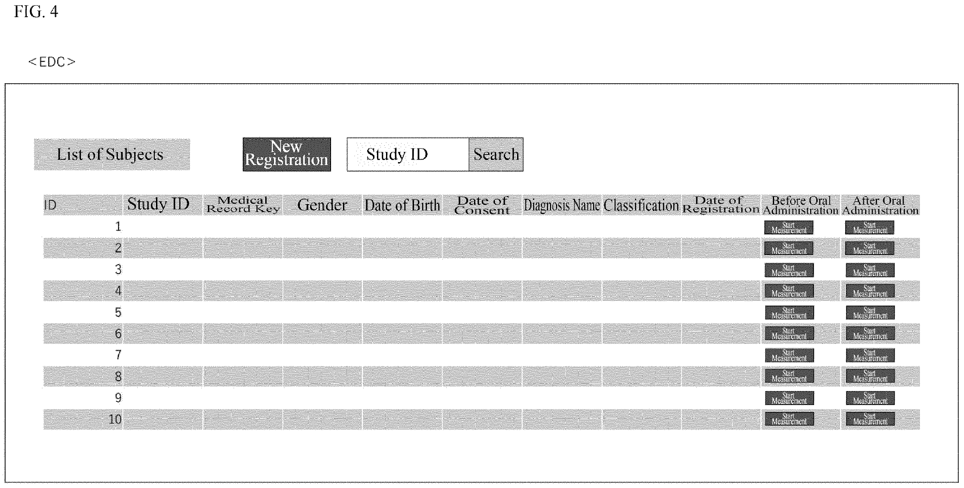

Next, the EDC system will be explained. FIG. 4 is a diagram illustrating an example of a screen displayed at the startup of the EDC system according to an embodiment of the invention. As illustrated in FIG. 4, the screen of the EDC system 23a displays a list of subjects by ID. The list includes the following items: study ID, medical record ID, gender, date of birth, date of consent, diagnosis name, classification, date and time of registration, and measurement tag (information about the measurement status, such as before oral administration, after oral administration, etc.).

A new subject can be registered by pressing or clicking the New Registration button illustrated in FIG. 4. For collecting data from a subject, the measurement can be started by selecting a Start Measurement button located on the measurement tag (indicating the measurement status: in this example, before and after oral administration) associated with the study ID of the subject.

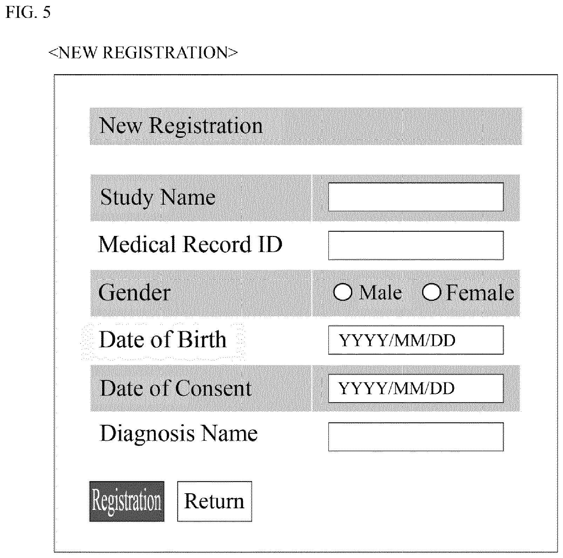

FIG. 5 is a diagram illustrating an example of a screen for newly registering a subject in the EDC system. As illustrated in FIG. 5, a subject can be newly registered by selecting or entering the study name (subject), medical record ID, gender, date of birth, date of consent, and diagnosis name. Once the registration is complete, a study ID associated with the subject is issued.

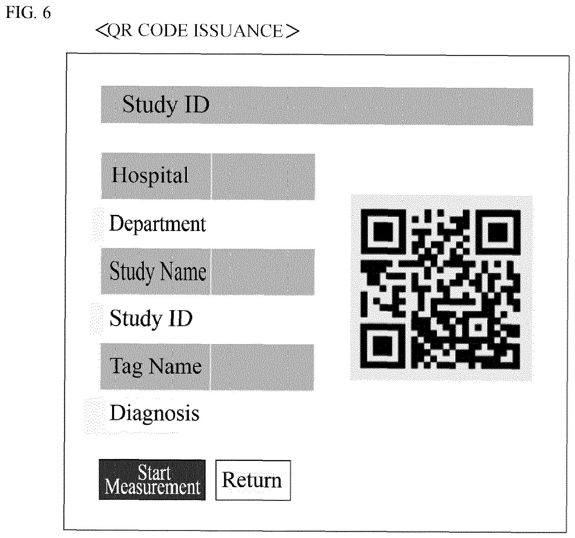

FIG. 6 is a diagram illustrating an example of a screen displayed when a QR code is issued. As illustrated in FIG. 6, the hospital name, medical department name, study name, study ID, tag name, and diagnosis name are displayed in addition to the study ID assigned to each patient. The above information is also output as a QR code. When the QR code is read by the dedicated application 21a on the smartphone master device 21, the information is transmitted to the smartphone master device 21 and the information on both sides is linked.

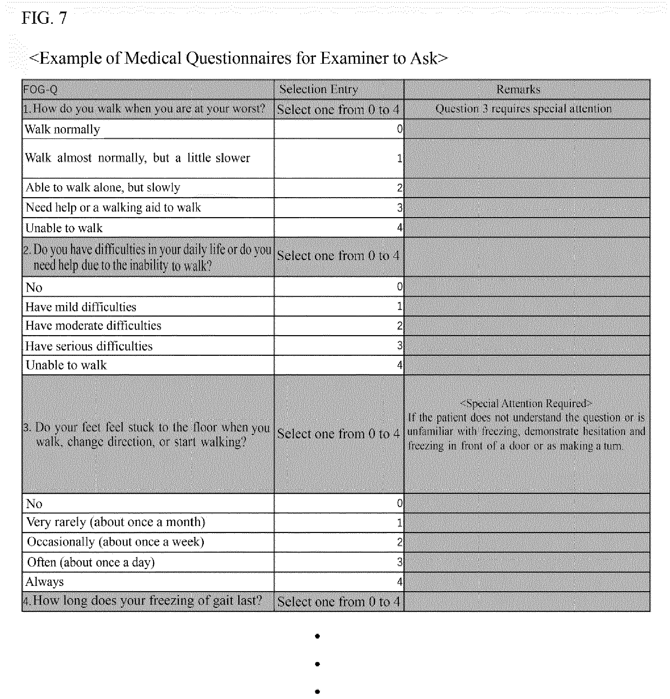

FIG. 7 is a diagram illustrating an example of data of medical questionnaires for the examiner to ask according to an embodiment of the invention. The medical questionnaire data is entered into the EDC system 23a in association with the study ID or the measurement tag.

As illustrated in FIG. 7, The medical questionnaire data includes a plurality of types of medical questionnaires (FOG-Q, etc.). The medical questionnaire data may be stored in advance in the memory 13 of the server 10, or it may be automatically generated by the server 10 according to each subject.

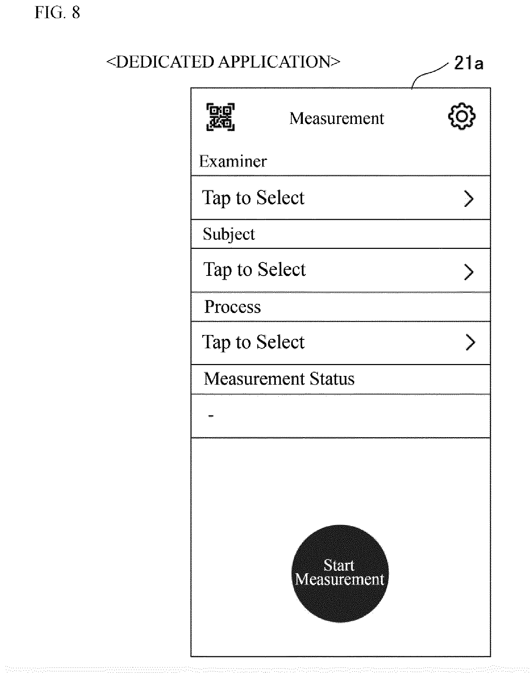

Next, the dedicated application 23a will be explained. FIG. 8 is a diagram illustrating an example of a screen displayed at the startup of the dedicated application on the examiner terminal according to an embodiment of the invention. As illustrated in FIG. 8, a QR code reader button is displayed in the upper left corner, which can be pressed or clicked to read a QR code displayed on the EDC system 23a.

The information read is displayed in columns for examiner information and subject information as described below. The examiner information includes a hospital name, medical department name, and study name, while the subject information includes a study ID, measurement tag, and diagnosis name.

By pressing a Start Measurement button after selecting a measurement process, the diagnosis prediction system 100 initiates the measurement and collection of biological data.

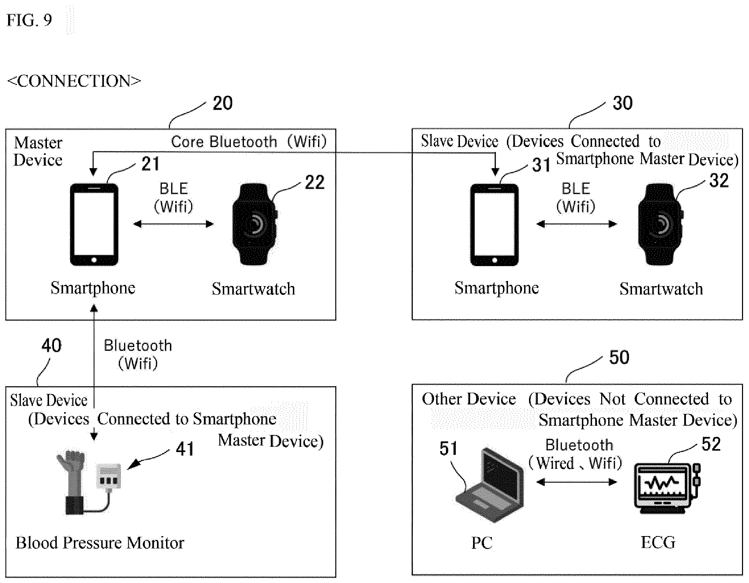

FIG. 9 is a diagram illustrating an example of a device connection between the master device system and the slave device system during measurement according to an embodiment of the invention.

Data is collected from the devices (the smartphone slave device 31, the blood pressure monitor 41) connected to the smartphone master device 21 via wireless communication such as Bluetooth (registered trademark) or Wi-Fi (registered trademark). The other device(s) 50 (the electrocardiograph 52), which is not connected to the smartphone master device 21, is connected to a terminal, such as the personal computer 23, to collect data therefrom.

In this example, the smartphone master device 21 is connected to the smartwatch master device 22, and the smartphone slave device 31 is connected to the smartwatch slave device 32.

There may be cases where there is no other device 50 that is not connected to the smartphone master device 21. In such a case, the functions of the smartphone master device or a device connected thereto replace the functions of the other device.

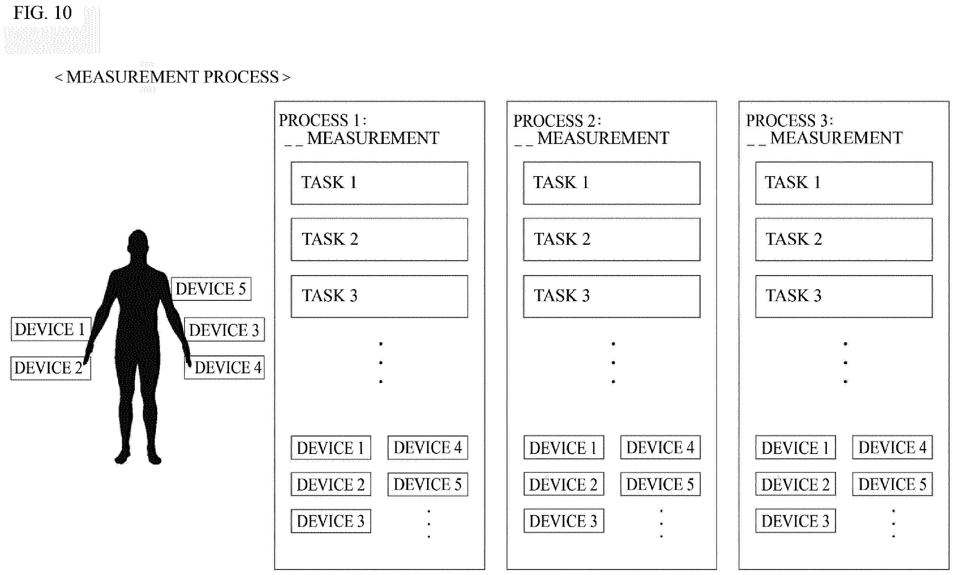

Next, the measurement processes will be described. FIG. 10 is a diagram illustrating the measurement processes according to an embodiment of the invention. The subject wears a plurality of digital devices 1 to 5 to measure biological data. Each of the measurement processes includes a plurality of tasks 1 to 3, and the digital devices are used in a series of tasks to measure and collect biological data all at once.

The measurement process is characterized by the use of a plurality of devices and the setting of a plurality of tasks. The measurement process 1 includes a plurality of tasks (task 1 to task 3, etc.) in which the subject wears a plurality of digital devices 1 to 5 and the like. The measurement process 2 includes a plurality of tasks (task 1 to task 3, etc.) in which the subject wears a plurality of digital devices 1 to 5 and the like. The measurement process 3 includes a plurality of tasks (task 1 to task 3, etc.) in which the subject wears a plurality of digital devices 1 to 5 and the like. Note that the number of digital devices and the number of tasks for each measurement process are provided as examples only and can be set as appropriate depending on the measurement content and accuracy.

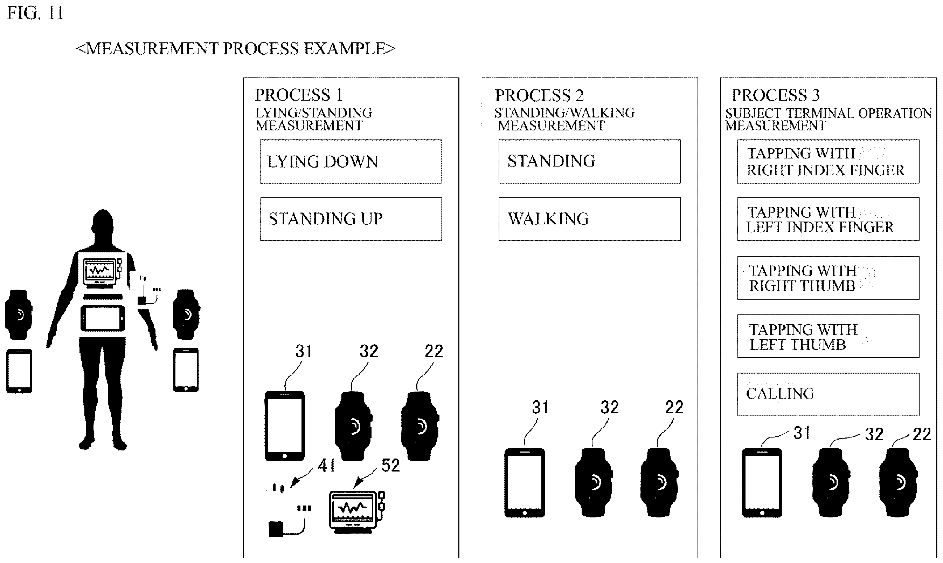

FIG. 11 is a diagram illustrating an example of the measurement processes according to an embodiment of the invention. FIG. 1 illustrates three example measurement processes 1 to 3. The measurement processes 1 to 3 include lying/standing measurement, standing/walking measurement, and subject terminal operation measurement, respectively.

In the measurement process 1, the lying/standing measurement includes a task of lying down and a task of standing up. In the measurement of the measurement process 1, the subject wears, as the plurality of digital devices, the smartphone slave device 31, the smartwatch slave device 32, the smartwatch master device 22, the blood pressure monitor 41, and the electrocardiograph 52.

In the measurement process 2, the standing/walking measurement includes a task of standing and a task of walking. In the measurement of the measurement process 2, the subject wears, as the plurality of digital devices, the smartphone slave device 31, the smartwatch slave device 32, and the smartwatch master device 22.

In the subject terminal operation measurement of the measurement process 3, the subject wears, as the plurality of digital devices, the smartphone slave device 31, the smartwatch slave device 32, and the smartwatch master device 22. In the measurement process 3, the subject terminal operation measurement includes a task of tapping with the right index finger, a task of tapping with the left index finger, a task of tapping with the right thumb, a task of tapping with the left thumb, and a task of calling.

In this manner, by setting a plurality of tasks in each of the measurement processes, biological data can be measured according to the movement of the subject and collected efficiently.

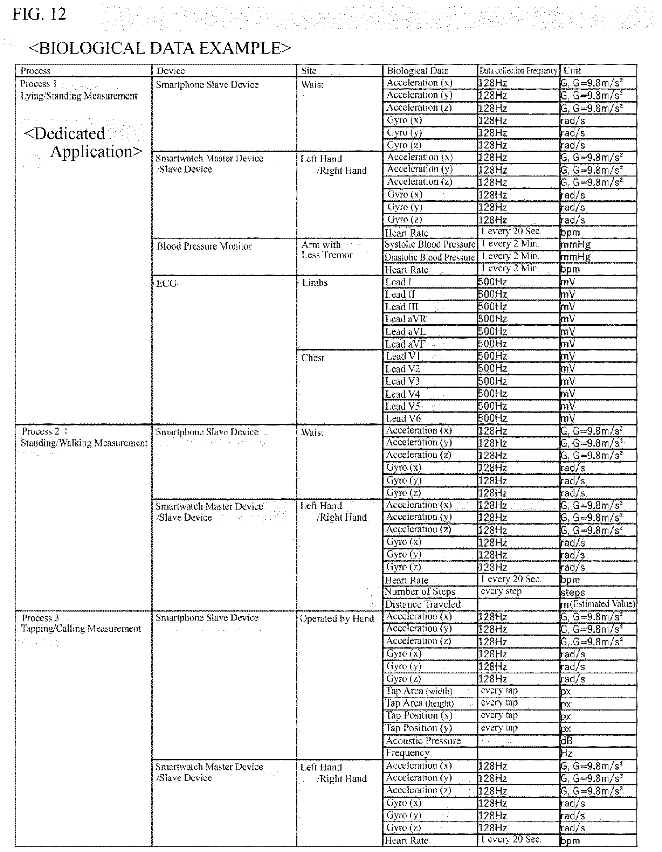

FIG. 12 is a diagram illustrating an example of a table of biological data collected in each measurement process. As illustrated in FIG. 12, the table of biological data includes the following items: process name, device, attachment site, biological data, data collection frequency, unit, and the like.

In the measurement of the measurement process 1, the subject wears the smartphone slave device 31 on the waist, the smartwatch master device 22 and the slave device 32 on the left or right hand (wrist), the blood pressure monitor 41 on the arm with less tremor, and the electrocardiograph 52 on the limbs and chest.

In the lying/standing measurement of the measurement process 1, the smartphone slave device 31 attached to the waist measures acceleration (x), acceleration (y), acceleration (z), gyro (x), gyro (y), and gyro (z) as biological data. The smartwatch master device 22 and the slave device 32 attached to the left/right hand measure acceleration (x), acceleration (y), acceleration (z), gyro (x), gyro (y), gyro (z), and heart rate as biological data. The blood pressure monitor 41 attached to the arm with less tremor measures systolic blood pressure, diastolic blood pressure, and heart rate as biological data. The electrocardiograph 52 attached to the limbs measures lead I, lead II, lead III, lead aVR, lead aVL, and lead aVF as biological data. The electrocardiograph 52 attached to the chest measures lead V1, lead V2, lead V3, lead V4, lead V5, and lead V6 as biological data.

In the standing/walking measurement of the measurement process 2, the smartphone slave device 31 attached to the waist measures acceleration (x), acceleration (y), acceleration (z), gyro (x), gyro (y), and gyro (z) as biological data. The smartwatch master device 22 and the slave device 32 attached to the left/right hand measure acceleration (x), acceleration (y), acceleration (z), gyro (x), gyro (y), gyro (z), heart rate, number of steps, and distance traveled as biological data.

In the subject terminal operation measurement of the measurement process 3, the smartphone slave device 31 operated by hand measures acceleration (x), acceleration (y), acceleration (z), gyro (x), gyro (y), gyro (z), tap area (width), tap area (height), tap position (x), tap position (y), sound pressure (acoustic pressure), and frequency as biological data. The smartwatch master device 22 and the slave device 32 attached to the left/right hand measure acceleration (x), acceleration (y), acceleration (z), gyro (x), gyro (y), gyro (z), and heart rate as biological data.

Biological data of the subject during the tasks can be measured and collected in this manner.

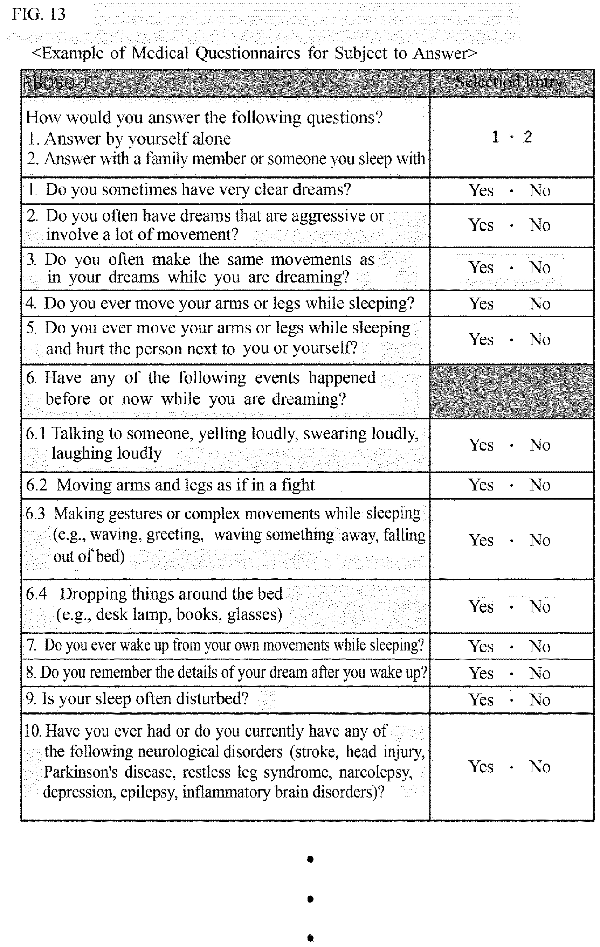

FIG. 13 is a diagram illustrating an example of data of medical questionnaires for the subject to answer according to an embodiment of the invention. The medical questionnaire data includes a plurality of types of medical questionnaires (RBDSQ-J, etc.). The medical questionnaire data is entered into the smartphone slave device 31 in association with the study ID or measurement tag.

The smartphone slave device 31 sends questionnaires and answers to the questionnaires as medical questionnaire data to the smartphone master device 21. The smartphone master device 21 sends the medical questionnaire data to the server 10, and the server 10 stores the medical questionnaire data.

Once the above is completed, the following data collection is completed.

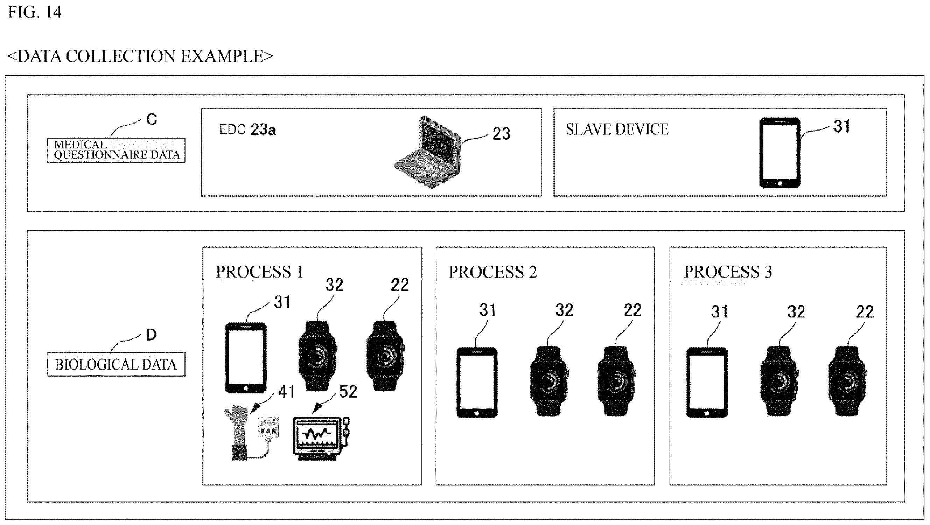

FIG. 14 is a diagram illustrating an example of data collection according to an embodiment of the invention.
1. Medical questionnaire data C provided by the examiner on the EDC system 23a
2. Medical questionnaire data C answered by the subject on the smartphone slave device 31
3. Biological data D measured and collected by the digital devices in the measurement processes 1 to 3

The medical questionnaire data C provided or asked by the examiner on the EDC system 23a is sent to the server 10 through the personal computer 23 and stored in the memory 13 of the server 10. The medical questionnaire data C answered by the subject on the smartphone slave device 31 is sent to the server 10 through the smartphone master device 21 and stored in the memory 13 of the server 10.

The biological data D measured and collected by the digital devices in the measurement processes 1 to 3 is sent to the server 10 through the smartphone master device 21 or the personal computer 23 and stored in the memory 13 of the server 10. Note that the medical questionnaire data and biological data collected on the smartphone slave device 31 can be sent directly to the server 10 without being collected on the smartphone master device 21.

FIG. 15 is an explanatory diagram illustrating a prediction model generated by the server according to an embodiment of the invention. As illustrated in FIG. 15, data on medical questionnaires E, acceleration and gyro F, heart rate and systolic/diastolic blood pressure G, electrocardiograph H, gait I, tap J, and voice K are obtained for a single measurement from the EDC system 23a, the smartphone slave device 23a, and the processes 1 to 3, as the medical questionnaire data C and the biological data D.

The input data 81 is obtained by performing preprocessing 82 to extract features from the data on medical questionnaires E, acceleration and gyro F, heart rate and systolic/diastolic blood pressure G, electrocardiograph H, gait I, tap J, and voice K. In addition, test scores, diagnosis name, time of onset, and clinical rating scale can be obtained for a single subject.

The former, i.e., the data obtained by performing the preprocessing 82 on the medical questionnaire data E and the biological data F to K, is used as the input data 81, and the latter, i.e., the test scores, diagnosis name, time of onset, and clinical rating scale, is used as the training data 90 to create data sets. The data sets are then divided into those for training, validation, and testing.

The techniques of the deep learning 91 and machine learning 92 are used to train a model and to validate and test learning results (step S30). A highly accurate model is extracted from the trained model and used as the prediction model 95 for test scores, diagnosis name, time of onset, and clinical rating scale.

As a specific example, the test scores may include early HMR, delayed HMR, or WR of ¹²³I-MIBG myocardial scintigraphy, which reflects the cardiac sympathetic status. The diagnosis name may be Parkinson's syndrome (Parkinson's disease, multiple system atrophy, progressive supranuclear palsy, corticobasal degeneration, cerebrovascular parkinsonism, drug-induced parkinsonism, normal pressure hydrocephalus) or normal. The time of onset may be the number of months from the present. The clinical rating scale may be the Hoehn-Yahr severity scale, UPDRS Part III, or mini-mental scale examination (MMSE).

Referring back to FIG. 15, the function of the server 10 as a learning device and a prediction model generation method will be described. The memory 13 stores biological data D measured by a digital device worn by each of a plurality of subjects, medical questionnaire data C corresponding to the biological data of the subjects, and disease-related information corresponding to each of the subjects such as test scores, diagnosis name, time of onset, or clinical rating scale (memory step).

The learning unit 14 generates and updates the trained prediction model 95 using the deep learning 91 in which the biological data F to K and the medical questionnaire data E of the subjects stored in the memory 13 are used as the input data 81, and the disease-related information corresponding to each of the subjects, such as test scores, diagnosis name, time of onset, or clinical rating scale, stored in the memory 13 is used as the training data 90 (generation/update step).

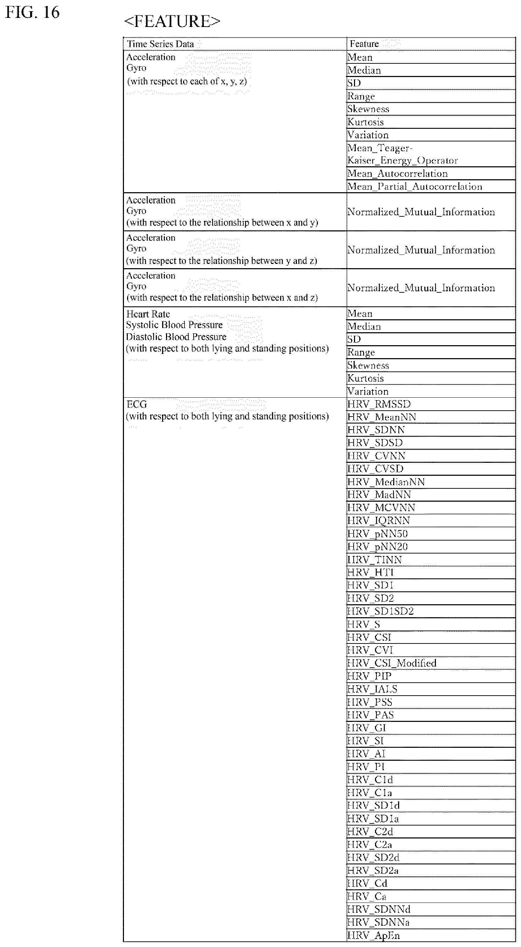

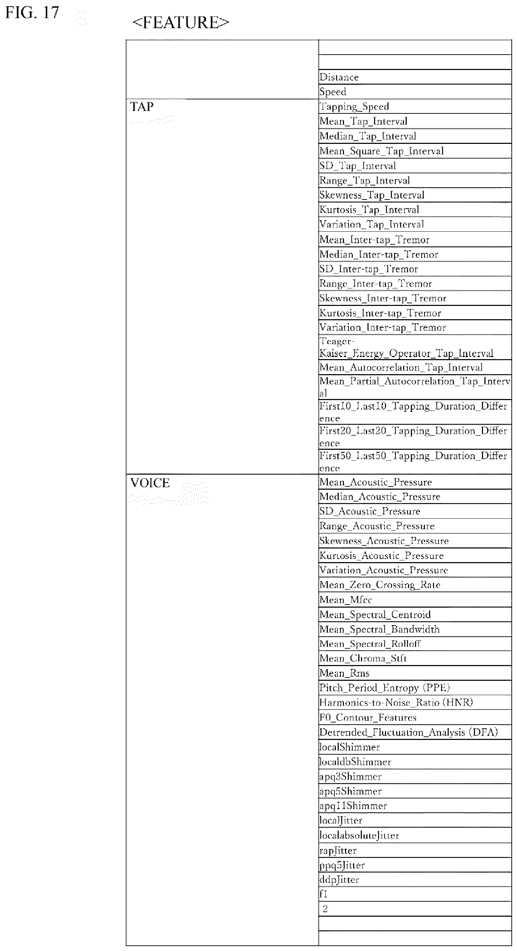

FIGS. 16 and 17 are a continuous diagram illustrating features extracted from the biological data. In the preprocessing 82, features are extracted from the biological data on acceleration and gyro F, heart rate and systolic/diastolic blood pressure G, electrocardiograph H, gait I, tap J, and voice K to generate the input data 81 of the biological data.

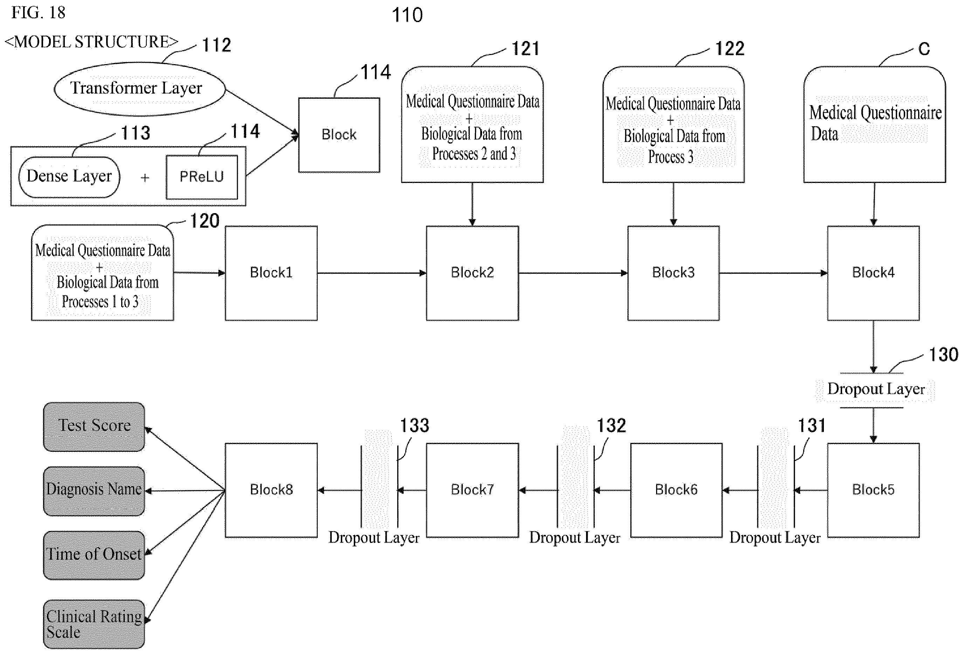

FIG. 18 is a diagram illustrating a model structure according to an embodiment of the invention. A transformer layer 112 or a combination of a dense layer 113 and a PReLU layer 114 was used for each block 111 (block 1 to block 8). Dropout layers 130 to 133 were provided between adjacent pairs of blocks 4 to 8, respectively.

A model was constructed using the following four patterns:
Pattern 1: The medical questionnaire data C was used to predict test scores, diagnosis name, time of onset, and clinical rating scale. The data was entered from block 1 of the network to predict the results through blocks 2 to 8.
Pattern 2: The medical questionnaire data and biological data from process 3 (122) were used to predict test scores, diagnosis name, time of onset, and clinical rating scale. The data was entered from block 2 of the network to predict the results through blocks 3 to 8.
Pattern 3: The medical questionnaire data and biological data from processes 2 and 3 (122) were used to predict test scores, diagnosis name, time of onset, and clinical rating scale. The data was entered from block 3 of the network to predict the results through blocks 4 to 8.
Pattern 4: The medical questionnaire data and biological data from processes 1 to 3 (120) were used to predict test scores, diagnosis name, time of onset, and clinical rating scale. The data was entered from block 4 of the network to predict the results through blocks 5 to 8.

Since two types of blocks were used to predict the four results in each pattern, a total of 32 (4 × 4 × 2) models were generated. If these are used as a single set of training data upon prediction of the four results, a total of 8 (4 × 2) models are generated.

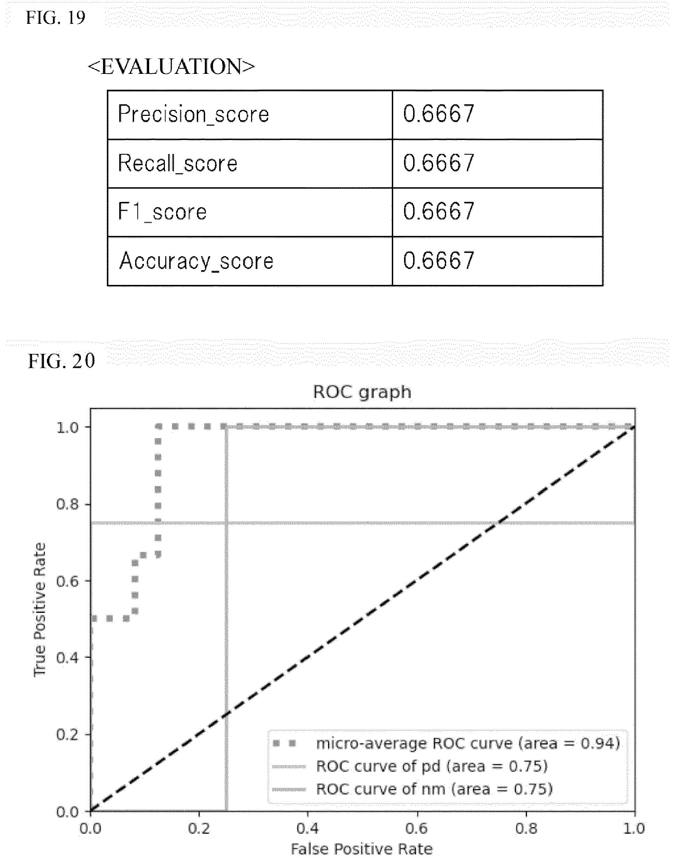

FIG. 19 is a diagram illustrating the evaluation of diagnosis name prediction results for the verification/test data of 6 cases.

A total of 1,162 feature data items generated from the medical questionnaire data and the biological data from processes 1 to 3, i.e., the input data 81, and diagnostic name data, i.e., the training data 90, were collected and combined into a single data set.

Of the data sets of 12 cases in total (6 cases of Parkinson's disease and 6 normal cases), 6 cases were used for training and 6 cases were used for verification/testing, and the diagnosis name was predicted by the prediction unit 12 of the server 10 using the generated prediction model 95.

A combination of a dense layer and a PReLU layer is used for the blocks of the model structure.

FIG. 20 is a graph illustrating the receiver operating characteristic (ROC) curves of the result of the diagnosis name prediction in the diagnosis prediction system according to an embodiment of the invention. As illustrated in FIG. 20, the prediction result is a vector of length 5, and the true value is also a vector of length 5. The ROC curve of pd represents the ROC with a determination of Parkinson's disease, while the ROC curve of nm represents the ROC with a determination of normal. The micro-average ROC curve represents the ROC when six vectors of length 5 are connected so that the prediction result is a vector of length 30 and the true value is a vector of length 30.

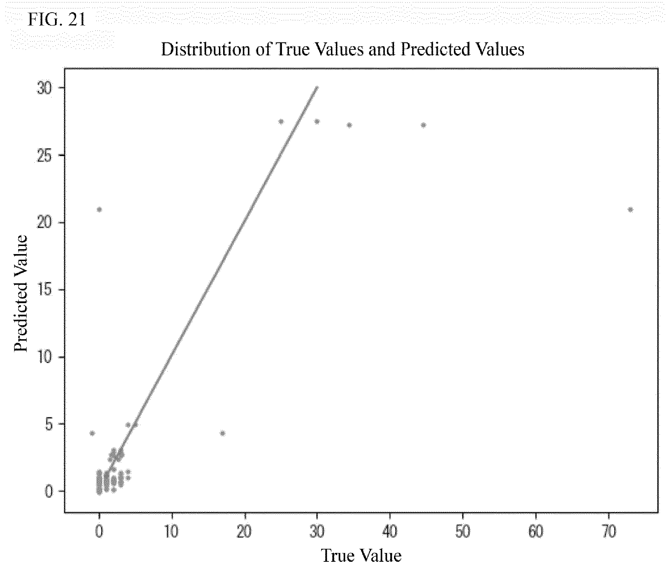

FIG. 21 is a graph illustrating the distribution of true values and predicted values obtained by an example of the prediction system according to an embodiment of the invention.

A total of 484 feature data items generated from the medical questionnaire data and the biological data from processes 2 and 3, i.e., the input data 81, and a total of 63 data items including test scores, diagnosis name, time of onset, and clinical rating scale, i.e., the training data 90, were collected and combined into a single data set.

Of the data sets of 12 cases in total (6 cases of Parkinson's disease and 6 normal cases), 8 cases were used for training, 2 cases were used for verification, and 2 cases were used for testing. The test scores, diagnosis name, time of onset, and clinical rating scale were predicted by the prediction unit 12 of the server 10 using the generated prediction model 95.

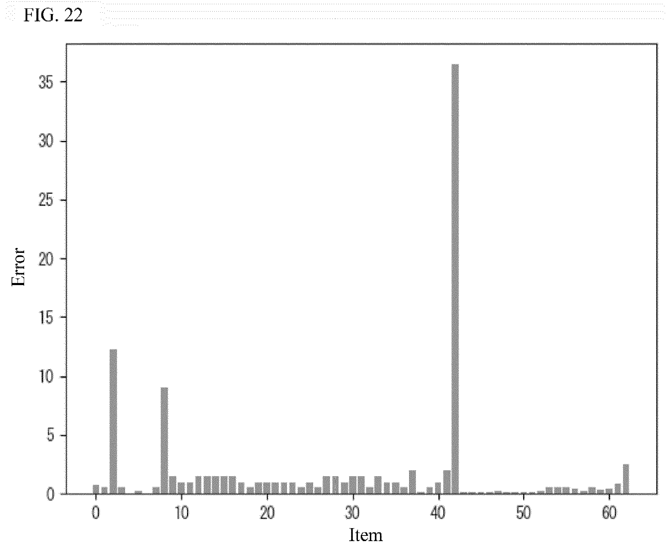

FIG. 22 is a graph illustrating the distribution of the mean absolute errors of the 63 items in the test data of 2 cases. The mean squared error of the 63 items in the samples of the 2 cases was 30.325128078460693. The maximum, minimum, mean, median, and standard deviation of the mean absolute errors were 36.480095863342285, 0.007834188640117645, 1.6707386628148102, 0.7177975177764893, 4.78676059867263, respectively.

Although specific embodiments of the invention have been described and illustrated, it is to be understood that the invention is not to be limited to the embodiments disclosed herein. Various changes, modifications, and alterations may be made within the scope of the invention as defined by the appended claims. Furthermore, all or some of the elements described in the above embodiments may be combined.

In the above embodiments, an example has been described in which the server 10 predicts the disease-related information of the subject; however, the disease-related information of the subject may also be predicted by the smartphone master device 21, the smartphone slave device 31, the personal computer 23, or a digital device. This can be realized by providing the smartphone master device 21, the smartphone slave device 31, the personal computer 23, or a digital device with the functions of the input unit 11, the prediction unit 12, the memory 13, and the like of the server 10. In addition, by installing the prediction model 95 generated by the server 10 in the server 10, the smartphone master device 21, the smartphone slave device 31, the personal computer 23, or a digital device, the diagnosis prediction system and the diagnosis prediction method can be implemented in the server 10, the smartphone master device 21, the smartphone slave device 31, the personal computer 23, or a digital device.

The diagnosis prediction method is implemented by an information processing device and can be performed by the server 10, the smartphone master device 21, the smartphone slave device 31, or the personal computer 23.

The diagnosis prediction method includes: an input step of receiving biological data D measured by a digital device worn by a subject and medical questionnaire data C of the subject; and a prediction step of predicting a test score, diagnosis name, time of onset, or clinical rating scale as disease-related information of the subject based on the biological data D and the medical questionnaire data C of the subject received in the input step by using the trained prediction model 95 that has undergone the deep learning 91, in which biological data D measured by a digital device worn by each of a plurality of subjects and medical questionnaire data C corresponding to the biological data of the subjects are used as the input data 81, and disease-related information corresponding to each of the subjects, such as a test score, diagnosis name, time of onset, or clinical rating scale, is used as the training data 90.

A computer program can be executed on an information processing device to predict the diagnosis. The computer program causes the information processing device to perform: an input step of receiving biological data D measured by a digital device worn by a subject and medical questionnaire data C of the subject; and a prediction step of predicting a test score, diagnosis name, time of onset, or clinical rating scale as disease-related information of the subject based on the biological data D and the medical questionnaire data C of the subject received in the input step by using the trained prediction model 95 generated by the deep learning 91, in which biological data D measured by a digital device worn by each of a plurality of subjects and medical questionnaire data C corresponding to the biological data D of the subjects are used as the input data 81, and disease-related information corresponding to each of the subjects, such as a test score, diagnosis name, time of onset, or clinical rating scale, is used as the training data 90.

The computer program is executed by the server 10, the smartphone master device 21, the smartphone slave device 31, or the personal computer 23.

### [List of Reference Signs]

- 100: Diagnosis Prediction System
- 10: Server
- 11: Input Unit
- 12: Prediction Unit
- 13: Memory
- 14: Learning Unit
- 21: Smart Phone Master device
- 22: Smartwatch Master device
- 23: Personal Computer
- 23a: EDC System
- 31: Smartphone Slave device
- 32: Smartwatch Slave device
- 41: Blood Pressure Monitor
- 51: Personal Computer
- 52: Electrocardiograph (ECG)

## Claims

1. A diagnosis prediction system, comprising:
an input unit that receives biological data measured by a digital device worn by a subject and medical questionnaire data of the subject; and
a prediction unit that predicts disease-related information of the subject based on the biological data and the medical questionnaire data of the subject received through the input unit by using a trained model that has undergone deep learning, in which biological data measured by a digital device worn by each of a plurality of subjects and medical questionnaire data corresponding to the biological data of the subjects are used as input data, and disease-related information corresponding to each of the subjects is used as training data.

2. The diagnosis prediction system according to claim 1, wherein the disease-related information includes at least one of the following: test score, diagnosis name, time of onset, and clinical rating scale.

3. The diagnosis prediction system according to claim 1, further comprising:
an examiner terminal operated by an examiner; and
a subject terminal operated by the subject,
wherein the examiner terminal or the subject terminal collects the biological data and the medical questionnaire data through the digital device.

4. The diagnosis prediction system according claim 1, further comprising:
an examiner terminal operated by an examiner; and
an information processing device including an electronic data capture (EDC) system, wherein
the information processing device issues code information that contains measurement-related information of the subject, and
the examiner terminal reads the code information so that the system authenticates the subject and the measurement-related information of the subject.

5. The diagnosis prediction system according to claim 1, wherein
measurement involves a plurality of digital devices each performing a series of measurements or a plurality of measurement processes each defining movements of the subject, and
each of the measurement processes includes a measurement in which the subject makes different movements.

6. The diagnosis prediction system according to claim 1, further comprising a selection means used to select a digital device for a series of measurements or to select a measurement process from measurement processes each defining movements of the subject,
wherein the biological data or the medical questionnaire data is collected according to the measurement process selected.

7. The diagnosis prediction system according to claim 1, wherein
the digital device includes a plurality of digital devices,
measurement involves a plurality of digital devices each performing a series of measurements or a plurality of measurement processes each defining movements of the subject,
each of the measurement processes includes a plurality of tasks, and
the biological data is collected by the digital devices according to a series of tasks of the plurality of tasks.

8. The diagnosis prediction system according to any one of claims 5 to 7, wherein the measurement processes include at least one of the following measurements: lying down and standing up, standing and walking, and operating a subject terminal.

9. A learning device comprising:
a memory that stores biological data measured by a digital device worn by each of a plurality of subjects, medical questionnaire data corresponding to the biological data of the subjects, and disease-related information corresponding to each of the subjects; and
a learning unit that generates and updates a trained model using deep learning, in which the biological data and the medical questionnaire data of the subjects stored in the memory are used as input data, and the disease-related information corresponding to each of the subjects stored in the memory is used as training data.

10. A computer program executed on an information processing device, the computer program causing the information processing device to perform:
an input step of receiving biological data measured by a digital device worn by a subject and medical questionnaire data of the subject; and
a prediction step of predicting disease-related information of the subject based on the biological data and the medical questionnaire data of the subject received in the input step by using a trained model that has undergone deep learning, in which biological data measured by a digital device worn by each of a plurality of subjects and medical questionnaire data corresponding to the biological data of the subjects are used as input data, and disease-related information corresponding to each of the subjects is used as training data.

11. A diagnosis prediction method implemented by an information processing device, the method comprising:
an input step of receiving biological data measured by a digital device worn by a subject and medical questionnaire data of the subject; and
a prediction step of predicting disease-related information of the subject based on the biological data and the medical questionnaire data of the subject received in the input step by using a trained model that has undergone deep learning, in which biological data measured by a digital device worn by each of a plurality of subjects and medical questionnaire data corresponding to the biological data of the subjects are used as input data, and disease-related information corresponding to each of the subjects is used as training data.

12. A prediction model generation/updating method, comprising:
a memory step of storing biological data measured by a digital device worn by each of a plurality of subjects, medical questionnaire data corresponding to the biological data of the subjects, and disease-related information corresponding to each of the subjects; and
a generation/update step of generating and updating a trained prediction model using deep learning, in which the biological data and the medical questionnaire data of the subjects stored in the memory step are used as input data, and the disease-related information corresponding to each of the subjects stored in the memory step is used as training data.
